# EUROPEAN PATENT SPECIFICATION

(11) **EP 2 793 951 B1**
(45) Date of publication and mention of the grant of the patent: **19.02.2020**
(21) Application number: 12806465.6
(22) Date of filing: 20.12.2012
(51) Int. Cl.: A61K 49/04

(54) **STABILIZATION OF X-RAY DIAGNOSTIC COMPOSITION**
STABILISIERUNG EINER RÖNTGENDIAGNOSTISCHEN ZUSAMMENSETZUNG
STABILISATION D'UNE COMPOSITION DIAGNOSTIQUE AUX RAYONS X

(30) Priority: 21.12.2011 NO 20111762
(43) Date of publication of application: 29.10.2014
(73) Proprietor: GE HEALTHCARE AS, 0401 Oslo (NO)
(72) Inventor: GLØGÅRD, Christian, N-0401 Oslo (NO); THANING, Mikkel, N-0401 Oslo (NO); CERVENKA, Jan, 1263 Oslo (NO)
(74) Representative: O'Brien, Dominic Paul
(86) International application number: PCT/EP2012/076261
(87) International publication number: WO 2013/092783

(56) References cited:
- WO-A1-2010/079201
- WO-A1-2011/051387
- WO-A1-2011/117236
- WO-A2-2009/008734
- SHUI L L ET AL: "The effect of Iopamidol on rheological properties of monoglyceride/water system", COLLOIDS AND SURFACES. A, PHYSICACHEMICAL AND ENGINEERING ASPECTS, ELSEVIER, AMSTERDAM, NL, vol. 256, no. 1, 1 April 2005 (2005-04-01) , pages 85-90, XP027803029, ISSN: 0927-7757 [retrieved on 2005-04-01]
- SARAGUSTY J ET AL: "Protective effects of iodixanol during bovine sperm cryopreservation", THERIOGENOLOGY, LOS ALTOS, CA, US, vol. 71, no. 9, 1 June 2009 (2009-06-01), pages 1425-1432, XP026087969, ISSN: 0093-691X, DOI: 10.1016/J.THERIOGENOLOGY.2009.01.019 [retrieved on 2009-03-19]

## Description

### Field of the Invention:

The present invention relates to a composition comprising a non-ionic X-ray contrast agent in a pharmaceutically acceptable carrier, and particularly to a supersaturated X-ray composition comprising X-ray contrast agents with a high dissolution temperature in water. Particularly, the invention provides such composition which is stable, and wherein crystallisation during storage is avoided. The X-ray contrast agent is Ioforminol. The invention further relates to a process for the preparation of such stable diagnostic X-ray composition.

### Background of the Invention:

All diagnostic imaging is based on the achievement of different signal levels from different structures within the body. Thus, in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. Moreover, the greater the contrast the smaller the body structures that may be visualized in the imaging procedures, i.e. increased contrast can lead to increased spatial resolution. The diagnostic quality of images is strongly dependent on the inherent noise level in the imaging procedure, and the ratio of the contrast level to the noise level can thus be seen to represent an effective diagnostic quality factor for diagnostic images. Achieving improvement in such a diagnostic quality factor has long been and still remains an important goal.

In techniques such as X-ray, one approach to improve the diagnostic quality factor has been to introduce contrast enhancing materials formulated as contrast media into the body region being imaged. Thus for X-ray, early examples of contrast agents were insoluble inorganic barium salts which enhanced X-ray attenuation in the body zones into which they distributed. For the last 50 years the field of X-ray contrast agents has been dominated by soluble iodine containing compounds. Commercial available contrast media containing iodinated contrast agents are usually classified as ionic monomers such as diatrizoate (marketed e.g. under the trade mark Gastrografen™), ionic dimers such as ioxaglate (marketed e.g. under the trade mark Hexabrix™), nonionic monomers such as iohexol (marketed e.g. under the trade mark Omnipaque™), iopamidol (marketed e.g. under the trade mark Isovue™), iomeprol (marketed e.g. under the trade mark Iomeron™) and the non-ionic dimer iodixanol (marketed under the trade mark Visipaque™). The clinical safety of iodinated X-ray contrast media has continuously been improved over the recent decades through development of new agents; from ionic monomers (Isopaque™) to non-ionic monomers (e.g. Omnipaque™) and non-ionic dimers (e.g. Visipaque™).

The utility of the contrast media is governed largely by its toxicity, by its diagnostic efficacy, by adverse effects it may have on the subject to which the contrast medium is administered, but also by the ease of production, storage and administration. The toxicity and adverse biological effects of a contrast medium are contributed to by the components of the formulation medium, i.e. of the diagnostic composition, e.g. the solvent or carrier as well as the contrast agent itself and its components such as ions for the ionic contrast agents and also by its metabolites.

The manufacture of non-ionic X-ray contrast media involves the production of the active pharmaceutical ingredient (API), i.e. the contrast agent prepared in the primary production, followed by the formulation into the drug product, herein denoted the X-ray composition, prepared in the secondary production. In the preparation of an X-ray composition, the contrast agent is admixed with additives, such as salts, optionally after dispersion in a physiologically tolerable carrier. The contrast agent has to be completely solved in the carrier when additives are included and the composition is prepared. A well-known process for preparing X-ray compositions includes heating the contrast agent in the carrier, such as water for injection, to ensure complete dissolution. For instance, for the contrast media Visipaque™ the secondary production process includes dissolution of the contrast agent iodixanol in water for injection and heating to about 98 °C. Heating at this temperature for an adequate period of time ensures that the contrast agent is completely dissolved.

However, different X-ray contrast agents have different solubility. For instance WO 2009/008734 of GE Healthcare AS discloses a new class of compounds and their use as X-ray contrast agents. The compounds are dimers containing two linked iodinated phenyl groups. Compound I, now called Ioforminol, falling within the formula I of WO2009/008734, has been found by the applicant to have particularly favourable properties. Ioforminol is supersaturated at the relevant storage conditions.

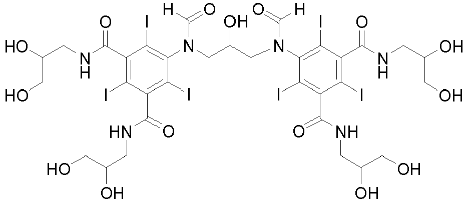

Compound I, Ioforminol: 5-[formyl-[3-[formyl-[3,5-bis(2,3-dihydroxypropylcarbamoyl)-2,4,6-triiodophenyl]amino]-2-hydroxypropyl]amino]-N,N'-bis(2,3-dihydroxypropyl)-2,4,6-triiodobenzene-1,3-dicarboxamide.

A solution in which the concentration of the solute (API) exceeds the equilibrium solute concentration at a given temperature is said to be supersaturated. This is possible because the solute does not precipitate immediately when the solution is cooled below the saturation temperature. Such solutions are denoted supersaturated.

As the solubility of Ioforminol decreases with decreasing temperature, the supersaturation increases. At room temperature the solubility of Ioforminol is limited. To achieve solutions with a concentration higher than the thermodynamic equilibrium concentration, at room temperature, Ioforminol is dissolved at a temperature above room temperature. When a clear solution has been achieved the solution is cooled and enters a state defined as supersaturated.

Supersaturated solutions are thermodynamically unstable and prone to nucleate and therefore to precipitate on storage. Among several factors, the onset of the precipitation depends on the degree of supersaturation, presence of the crystals of the solute and foreign particles such as dust or other impurities, i.e. purity, and storage temperature of the solution.

The injection solution of Ioforminol, i.e. the administrable X-ray composition, is highly supersaturated. The nucleation (precipitation) in the injection solution at storage conditions is strongly undesirable. The physical stability of the solution, i.e. prevention of the nucleation for a certain time at storage conditions, may be improved substantially by heat treatment of the solution well above its saturation temperature for a sufficiently long period of time. WO2011/117236 of the applicant is directed to a process involving hea treatment at low pH to avoid degradation and precipitation of an X-ray contrast agent composition. However, a high heat load is needed to obtain a seed-free solution. This heat load causes a greater degradation of the product and a lower pH in the final product resulting in liberation of iodine. This sets a restriction to the total heat load that may be given to the formulated solution. To overcome this challenge, alternative methods of stabilising the composition comprising Ioforminol have been investigated.

### Summary of the invention:

Therefore, a supersaturated stable X-ray composition comprising an X-ray contrast agent with a high dissolution temperature, and wherein crystallisation during storage is avoided, has been sought. It has surprisingly been found that if such composition comprising an X-ray contrast agent further includes a second compound which is structurally related to the contrast agent, the composition is kept stable and crystallisation is avoided during storing.

Thus, in a first aspect the invention provides a composition according to the claims comprising the contrast agent Ioforminol and a second compound with structural similarity with the contrast agent, functioning as an inhibitor of primary nucleation or crystal growth. Hence, the inhibitor stabilizes the Ioforminol composition.

### Detailed Description of the Preferred Embodiments:

The use of inhibitors of primary nucleation or crystal growth has been found to be a feasible and effective solution to the crystallisation problem. The Ioforminol composition e.g. at the concentration 320 mg I/ml, (∼50 mass%) is highly supersaturated at room (or storage) temperature. In the case of secondary production of ioforminol, using water for injection as the solvent, the dissolution temperature within the concentration range of e.g. 270-380 mg I/ml will differ only a few degrees from 110 °C. This means, that the system is unstable in thermodynamic terms and thus presupposed to crystallize. Stability of such a system is then only dependent on kinetics of the crystallization. To prevent crystallization, it is required to remove or deactivate all crystalline or heterogeneous nuclei from the solution, prevent or at least minimize primary nucleation and prevent growth of the eventually formed crystalline nuclei. It has been found that this can be achieved by including an inhibitor of primary nucleation or crystal growth in the Ioforminol composition.

There are several mechanisms of the nucleation- and growth inhibition, and the main groups are:
- Change of structure of the solution, hence an interaction between the solvents or solvent and solute, braking structures.
- Interaction with the solute molecules changing their ability to aggregate into the supercritical size.
- Interactions preventing the clusters from collapsing into the highly organised crystalline nuclei (entropy reduction).
- Adsorption on nuclei, causing increased energy need to further growth or secondary nucleate, and increasing probability of dissolution of the nuclei.
- Changing the interfacial tension, which is the important factor in nucleation as well as growth.
- Selective adsorption on growing surfaces, preventing further building of the crystalline lattice.

The contrast agent compound to be stabilized, Ioforminol, is the main compound of the composition, by weight. In one embodiment this is present in the composition in an amount of 85-99.99 weight%. More preferably the weight% of Ioforminol is 85-99 weight% and most preferably 85-95 weight%. The composition should comprise the inhibitor in an amount sufficient for the inhibitor to inhibit any nucleation or crystal growth, providing a stable solution, but not as much that it significantly changes other properties of the composition, such as e.g. the viscosity or the osmolality.

The amount needed will depend on which inhibitor is chosen. The inhibitor could e.g. be present in the composition in an amount from 0.1 to 15 weight%, such as 1 to 15 weight %, more preferably in an amount of 5-15 weight % and most preferably 10-15 weight%.

In addition to stabilising the composition the inhibitor added to the composition must meet certain requirements of tolerability and stability. To sufficiently act as an inhibitor of nucleation and crystal growth, acting according to any of the above mentioned mechanisms, it has been found that the inhibitor should have a chemical structure wherein at least an element of the chemical structure, such as a building block, a part or a component, is similar or identical with the chemical structure of the contrast agent, i.e. with Ioforminol. Accordingly, other elements of the structure of the inhibitor differ from the structure of the contrast agent. Such an inhibitor comprises a compound which is a non-ionic iodinated dimeric compound. It should be understood that the effectiveness of a particular inhibitor can only be assessed by empirical means.

The inhibitor comprises a dimeric compound comprising two linked triiodinated phenyl groups, of formula (I)

R-N(R6) -X-N(R6)-R Formula (I)

wherein
X denotes a C3 to C8 straight or branched alkylene moiety optionally with one or two CH₂ moieties replaced by oxygen atoms, sulphur atoms or NR4 groups and wherein the alkylene moiety optionally is substituted by up to six -OR4 groups;
R4 denotes a hydrogen atom or a C1 to C4 straight or branched alkyl group;
R6 denotes a hydrogen atom or an acyl function; and
each R independently is the same or different and denotes a 2,4,6-triiodinated phenyl group, further substituted by two groups R5 wherein each R5 is the same or different and denotes a hydrogen atom or a non-ionic hydrophilic moiety, provided that at least one R5 group in the compound of formula (I) is a hydrophilic moiety.

X preferably denotes a straight C3 to C8 alkylene chain optionally substituted by one to six -OR4 groups. More preferably X denotes a straight C3 to C5 alkylene chain having at least one -OR4 group, preferably with at least one hydroxyl group in a position that is not vicinal to the bridge nitrogen atom. More preferably the alkylene chain is substituted by one to three hydroxyl groups and still more preferably the alkylene chain is a straight propylene, butylene or pentylene chain substituted by one, two or three hydroxyl groups. Particularly preferred groups X are selected from 2-hydroxy propylene, 2,3-dihydroxy butylene, 2,4-dihydroxy pentylene and 2,3,4-trihydroxy pentylene, and most particularly X is the 2-hydroxy propylene entity.

R4 preferably denotes a hydrogen atom or a methyl group, most preferably a hydrogen atom.

The R6 substituents may be the same or different and preferably R6 denotes a hydrogen atom or a residue of an aliphatic organic acid, and in particular a C1 to C5 organic acid such as formyl, acetyl, propionyl, butyryl, isobutyryl and valeriyl moieties. Hydroxylated and metoxylated acyl moieties are also feasible. In a particularly preferred embodiment one R6 group in the compound of formula (I) denotes a formyl moiety and one denotes the acetyl moiety, or both R6 groups denote acetyl.

Each of the iodinated R groups denote a 2,4,6-triiodinated phenyl group, which can be further substituted by two groups R5 in the remaining 3 and 5 positions in the phenyl moiety. The non-ionic hydrophilic moieties, R5, may be any of the non-ionizing groups conventionally used to enhance water solubility. Hence, the R5 substituents may be the same or different and shall preferably all denote a non-ionic hydrophilic moiety comprising esters, amides and amine moieties, optionally further substituted by a straight chain or branched chain C1-10 alkyl groups, preferably C1-5 alkyl groups, where the alkyl groups also may have one or more CH₂ or CH moieties replaced by oxygen or nitrogen atoms. The R5 substituents may also further contain one or more groups selected from oxo, hydroxyl, amino or carboxyl derivative, and oxo substituted sulphur and phosphorus atoms. Each of the straight or branched alkyl groups preferably contains 1 to 6 hydroxy groups and more preferably 1 to 3 hydroxy groups. Therefore, in a further preferred aspect, the R5 substituents are the same or different and are polyhydroxy C1-5 alkyl, hydroxyalkoxyalkyl with 1 to 5 carbon atoms and hydroxypolyalkoxyalkyl with 1 to 5 carbon atoms, and are attached to the iodinated phenyl group via an amide or a carbamoyl linkage, preferably amide linkages.

The R5 groups of the formulas listed below are particularly preferred:
- CONH₂,
- CONHCH₃,
- CONH-CH₂-CH₂-OH,
- CONH-CH₂-CH₂-OCH₃,
- CONH-CH₂-CHOH-CH₂-OH,
- CONH-CH₂-CHOCH₃-CH₂-OH,
- CONH-CH₂-CHOH-CH2-OCH₃,
- CON(CH₃)CH₂-CHOH-CH₂OH,
- CONH-CH-(CH₂-OH)₂,
- CON-(CH₂-CH₂-OH)₂,
- CON-(CH₂-CHOH-CH₂-OH)₂,
- CONH-OCH₃,
- CON (CH₂-CHOH-CH₂-OH) (CH₂-CH₂-OH),
- CONH-C(CH₂-OH)₂ CH₃,
- CONH-C(CH₂-OH)₃, and
- CONH-CH(CH2-OH)(CHOH-CH2-OH),
- NH(COCH₃),
- N(COCH₃) C1-3 alkyl,
- N(COCH₃) - mono, bis or tris-hydroxy C1-4 alkyl,
- N(COCH₂OH) - hydrogen, mono, bis or tris-hydroxy C1-4 alkyl,
- N(CO-CHOH-CH₂OH) - hydrogen, mono, bis or trihydroxylated C1-4 alkyl,
- N(CO-CHOH-CHOH-CH₂OH) - hydrogen, mono, bis or trihydroxylated C1-4 alkyl,
- N(CO-CH-(CH₂OH)₂) - hydrogen, mono, bis or trihydroxylated C1-4 alkyl; and
- N(COCH₂OH)₂.

Even more preferably the R5 groups will be equal or different and denote one or more moieties of the formulas -CONH-CH₂-CH₂-OH, -CONH-CH₂-CHOH-CH₂-OH, -CON(CH₃)CH₂-CHOH-CH₂OH, -CONH-CH-(CH₂-OH)₂ and -CON-(CH₂-CH₂-OH)₂. Still more preferably both R groups are the same and the R2 groups in each R are the same or different and denote -CONH-CH₂-CH₂-OH, -CONH-CH₂-CHOH-CH₂-OH, CON(CH₃)CH2-CHOH-CH2OH, - CON-(CH₂-CH2-OH)₂ and -CONH-CH-(CH₂-OH)₂. In a particularly preferred embodiment, both R groups are the same and all R5 groups denote the entity of formula -CONH-CH₂-CHOH-CH₂-OH.

In a preferred embodiment, the inhibitor is the dimeric compound Iodixanol or the Compound II shown below, or mixures thereof. Compound II can been seen as a related impurity to Ioforminol, as it is a dimer structurally very similar to Ioforminol, but rather than having two formyl groups attached to the 2-hydroxypropane-1,3-diyl bridge, it has one formyl group and one acetyl group. It can be seen as half Iodixanol and half Ioforminol.

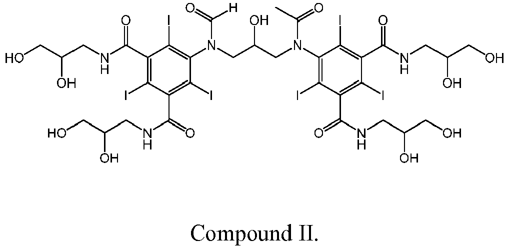

The diagnostic composition of the invention preferably includes excipients and additives, such as salts. Adverse effects of non-ionic contrast media can be reduced by the inclusion of metal ions such as sodium and calcium ions in the diagnostic composition. The sodium compound and the calcium compound of the composition may be provided in the form of salts, i.e. the compounds include physiologically tolerable counter ions, e.g. selected from the group of chloride, sulphate, phosphate and hydrogen carbonate. Preferably, the sodium compound is sodium chloride and the calcium compound is calcium chloride. The contrast agent is hence formulated with conventional carriers and excipients to produce a diagnostic composition. In addition to plasma ions, such as sodium and calcium ions, dissolved oxygen may be included. Further, chelating agents such as EDTA (ethylenediaminetetraacetic acid) or DTPA (diethylene triamine pentaacetic acid) may be included in the prepared composition to sequester metal ions from the solution. EDTA is preferred.

In a further aspect, the invention provides use of an inhibitor of primary nucleation or crystal growth in a method of increasing the stability of a composition comprising a contrast agent that has a low solubility, by including in the composition an inhibitor of primary nucleation or crystal growth. As for the first aspect, the contrast agent is Ioforminol. The type and amount of inhibitor to be used in the method is selected according to the first aspect of the invention.

In yet another aspect, the invention provides use of an inhibitor of primary nucleation or crystal growth in a process for preparation of a stable composition comprising the contrast agent Ioforminol in a carrier, wherein the process includes a step of including, such as adding, an inhibitor of primary nucleation or crystal growth in the Ioforminol composition. The type and amount of inhibitor to be used in the process is selected according to the first aspect of the invention. Ioforminol and the inhibitor can be combined in a composition by means of several methods. Either appropriate amounts of the two solid compounds are combined and subsequently dissolved in the carrier by necessary means, or two previously prepared solutions of the compounds are combined in suitable proportions. The following treatment of the composition follows established procedures for obtaining sterile solutions with appropriate salt balance and physiologically acceptable pH.

Accordingly, the process of the invention comprises the steps of either
a) Combining Ioforminol powder and powder of an inhibitor of primary nucleation or crystal growth, into a mixed powder;
   Dissolving the mixed powder in a carrier and mixing and heating to complete dissolution; or
b) Solving Ioforminol powder in a carrier to provide a first composition;
   Solving an inhibitor of primary nucleation or crystal growth in a carrier to provide a second composition;
   Mixing the first and second compositions and heating to complete dissolution,
   or
c) Heating a composition of Ioforminol in a carrier,
   Adding powder of an inhibitor of primary nucleation or crystal growth to the composition of Ioforminol and mixing and heating to complete dissolution.

The carrier is preferably a pharmaceutically acceptable carrier, and preferably an aqueous solution, preferably pure water.

The amount of inhibitor needed, and which inhibitor to include, should be selected as outlined in the first aspect.

To solve the Ioforminol powder completely the composition should be heated to e.g. 60-135 °C for as long as it takes to achieve complete dissolution, such as e.g. for 20-240 minutes.

In addition to the steps described, the process of the invention may further include the steps of:
- Mixing the components, i.e. the carrier, the contrast agent, the inhibitor and optional additives to completely dissolve the contrast agent in the carrier. Mixing means may be used and the mixing may be carried out by several mechanical mixing methods well known in the art, such as stirring in a mixing tank, using a static mixer or a mixing reactor.
- Filtration of the diagnostic composition, such as by micro- or ultrafiltration. The filtration is performed to remove and reduce in quantity particles, particularly particles with a size above a certain limit, e.g. above 10 000 Daltons, and/or for removal of endotoxins, which have survived the heat treatment.
- Dilution, i.e. diluting the composition to a concentration as desired.
- Filling, capsling and labeling.
- Final heat treatment: After filling the stabilized composition of the invention into bottles, these are preferably sterilized by autoclaving. Such a final heat treatment, e.g. by steam sterilization of the filled and sealed bottles at a suitable temperature, above the saturation temperature of the contrast agent, is critical with respect of dissolving foreign particles brought to the bottles by dust and to deactivate any insoluble foreign particles present in the solution.

Ioforminol can be prepared as outlined in WO 2009/008734. A general procedure is outlined on pages 16-20, and a specific method for preparation is provided in Example 1 of WO 2009/008734.

The diagnostic composition prepared by the process of the invention is in a ready to use concentration. Generally compositions in a ready to use form will have iodine concentrations of at least 100 mg I/ml, such as at least 150 mg I/ml, or with concentrations of at least 300 mg I/ml, e.g. 320 mg I/ml, or even 350, 360 or 400 mg I/ml, but also lower concentrations can be relevant.

The diagnostic composition of the invention is preferably for use in X-ray diagnosis. The composition may be administered as a bolus injection or by infusion. Further, the composition may be administered by intravascular, intravenous or intra-arterial administration. Alternatively, the composition may also be administered orally.

### Examples:

### Example 1. Stabilisation of Ioforminol with Iodixanol

Formulated Ioforminol (320 mg I/ml) and Visipaque (Iodixanol 320 mg I/ml) were filled on 100 ml glass vials in mixtures with ratios according to Table 1. The filling was performed by using a Flexicon PF5 peristaltic pump. The vials were sealed by closures comprising stoppers and overlying caps. The vials were split into two populations, one that was subjected to heat steam sterilisation at 121 °C (autoclaving) for 20 minutes and one that was used without further treatment.

**Table 1. Mixtures of formulated Ioforminol and Visipaque used for physical stability study w/w %.**

| w/w % | |
|---|---|
| Formulated Ioforminol (320 mg/ml) | Visipaque (320 mgl/ml) |
| 100 (Reference sample) | 0 |
| 95 | 5 |
| 85 | 15 |
| 75 | 25 |
| 50 | 50 |

### Physical stability study:

The autoclaved and non-autoclaved samples of different mixtures were placed on storage at 25, 40 and 60 °C respectively. The samples were subjected to visual inspection weekly for the first 5 weeks before inspection was carried out monthly.

For autoclaved samples stored at 60 °C and 40 °C crystallisation of the reference samples started after 1 and 8 weeks, respectively. No crystallisation was observed for the reference samples or mixtures containing Visipaque stored at 25 °C. For autoclaved samples the first crystallisation at 60 °C was observed for the mixture containing 5% Visipaque after 4 weeks while the corresponding crystallisation for 40 °C was detected after 6 months. No crystallisation was observed for the mixture containing 15% Visipaque regardless of the storage temperature.

For non-autoclaved samples only a minor effect was observed.

In summary, mixtures of Ioforminol with 5-15% Visipaque or more is showing a significant delay in crystallisation for autoclaved samples. This indicates that the Iodixanol (API in Visipaque) is a potent inhibitor of primary nucleation and hence is able to prevent crystallisation of Ioforminol.

## Claims

1. A composition comprising the contrast agent Ioforminol and further comprising a second compound functioning as an inhibitor of primary nucleation or crystal growth, wherein this inhibitor comprises a non-ionic iodinated dimeric compound wherein the inhibitor comprises a compound of formula (I)
R-N(R6) -X-N(R6)-R Formula (I)
wherein
X denotes a C3 to C8 straight or branched alkylene moiety optionally with one or two CH₂ moieties replaced by oxygen atoms, sulphur atoms or NR4 groups and wherein the alkylene moiety optionally is substituted by up to six -OR4 groups;
R4 denotes a hydrogen atom or a C1 to C4 straight or branched alkyl group;
R6 denotes a hydrogen atom or an acyl function; and
each R independently is the same or different and denotes a 2,4,6-triiodinated phenyl group, further substituted by two groups R5 wherein each R5 is the same or different and denotes a hydrogen atom or a non-ionic hydrophilic moiety, provided that at least one R5 group is a hydrophilic moiety.

2. A composition as claimed in claim 1 wherein the inhibitor is present in the composition in an amount from 0.1 weight% to 15 weight%.

3. A composition as claimed in claim 1 or 2 wherein the X group denotes an alkylene chain selected from propylene, butylene and pentylene substituted by one, two or three hydroxyl groups.

4. A composition as claimed in any of claims 1 to 3 wherein the inhibitor comprises the dimeric compound Iodixanol or the Compound II

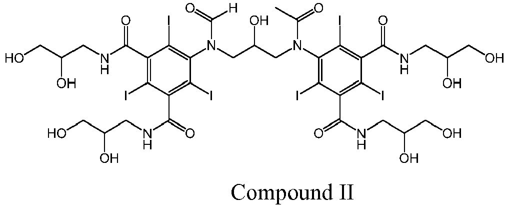

5. Use of an inhibitor of primary nucleation or crystal growth in a method of increasing the stability of a composition comprising Ioforminol, by including in the composition said inhibitor which comprises a non-ionic iodinated dimeric compound of formula (I)
R-N(R6)-X-N(R6)-R Formula (I)
wherein
X denotes a C3 to C8 straight or branched alkylene moiety optionally with one or two CH₂ moieties replaced by oxygen atoms, sulphur atoms or NR4 groups and wherein the alkylene moiety optionally is substituted by up to six -OR4 groups;
R4 denotes a hydrogen atom or a C1 to C4 straight or branched alkyl group;
R6 denotes a hydrogen atom or an acyl function; and
each R independently is the same or different and denotes a 2,4,6-triiodinated phenyl group, further substituted by two groups R5 wherein each R5 is the same or different and denotes a hydrogen atom or a non-ionic hydrophilic moiety, provided that at least one R5 group is a hydrophilic moiety.

6. Use of an inhibitor of primary nucleation or crystal growth in a process for preparation of a stable composition comprising the contrast agent Ioforminol in a carrier, wherein the process includes a step of including said inhibitor in the composition, and wherein the inhibitor comprises a non-ionic iodinated dimeric compound of formula (I)
R-N(R6) -X-N(R6)-R Formula (I)
wherein
X denotes a C3 to C8 straight or branched alkylene moiety optionally with one or two CH₂ moieties replaced by oxygen atoms, sulphur atoms or NR4 groups and wherein the alkylene moiety optionally is substituted by up to six -OR4 groups;
R4 denotes a hydrogen atom or a C1 to C4 straight or branched alkyl group;
R6 denotes a hydrogen atom or an acyl function; and
each R independently is the same or different and denotes a 2,4,6-triiodinated phenyl group, further substituted by two groups R5 wherein each R5 is the same or different and denotes a hydrogen atom or a non-ionic hydrophilic moiety, provided that at least one R5 group is a hydrophilic moiety.

7. The use of claim 6 wherein said process comprises the steps of either
a) combining Ioforminol powder and powder of an inhibitor of primary nucleation or crystal growth, into a mixed powder;
dissolving the mixed powder in a carrier and mixing and heating to complete dissolution; or
solving Ioforminol powder in a carrier to provide a first composition;
solving an inhibitor of primary nucleation or crystal growth in a carrier to provide a second composition;
mixing the first and second compositions and heating to complete dissolution;
or
heating a composition of Ioforminol in a carrier,
adding a powder of an inhibitor of primary nucleation or crystal growth to the composition of Ioforminol and mixing and heating to complete dissolution.

8. The use of claim 6 or 7 wherein said process further comprises any of the steps of filtration, dilution, filling, capsling and labeling, and heat treatment after filling.

9. The use of any of claims 6 to 8 wherein said inhibitor has a chemical structure wherein at least an element of the chemical structure is similar or identical with Ioforminol.

10. The use of any of the claims 6 to 9 wherein said inhibitor comprises the dimeric compound Iodixanol or the Compound II

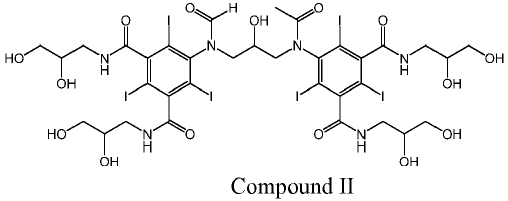

11. The use of any of the claims 6 to 10 wherein said inhibitor is present in the composition in an amount from 0.1 weight% to 15 weight%.

## Patentansprüche

1. Zusammensetzung, umfassend das Kontrastmittel loforminol und weiter umfassend eine zweite Verbindung, die als Inhibitor der primären Keimbildung oder des Kristallwachstums fungiert, wobei dieser Inhibitor eine nichtionische iodierte dimere Verbindung umfasst, wobei der Inhibitor eine Verbindung der Formel (I) umfasst
R-N(R6)-X-N(R6)-R Formel (I)
wobei
X einen geraden oder verzweigten C3- bis C8-Alkylenteil bezeichnet, wobei optional ein oder zwei CH₂-Teile durch Sauerstoffatome, Schwefelatome oder NR4-Gruppen ersetzt sind und wobei der Alkylenteil optional durch bis zu sechs -OR4-Gruppen substituiert ist;
R4 ein Wasserstoffatom oder eine gerade oder verzweigte C1- bis C4-Alkylgruppe bezeichnet;
R6 ein Wasserstoffatom oder eine Acylfunktion bezeichnet; und
jedes R unabhängig voneinander gleich oder verschieden ist und eine 2,4,6-triiodierte Phenylgruppe bezeichnet, weiter substituiert durch zwei Gruppen R5, wobei jede R5 gleich oder verschieden ist und ein Wasserstoffatom oder einen nichtionischen hydrophilen Teil bezeichnet, vorausgesetzt, dass mindestens eine R5-Gruppe ein hydrophiler Teil ist.

2. Zusammensetzung nach Anspruch 1, wobei der Inhibitor in der Zusammensetzung in einer Menge von 0,1 Gew.-% bis 15 Gew.-% vorhanden ist.

3. Zusammensetzung nach Anspruch 1 oder 2, wobei die X-Gruppe eine Alkylenkette bezeichnet, ausgewählt aus Propylen, Butylen und Pentylen, substituiert durch eine, zwei oder drei Hydroxylgruppen.

4. Zusammensetzung nach einem der Ansprüche 1 bis 3, wobei der Inhibitor die dimere Verbindung lodixanol oder die Verbindung II umfasst

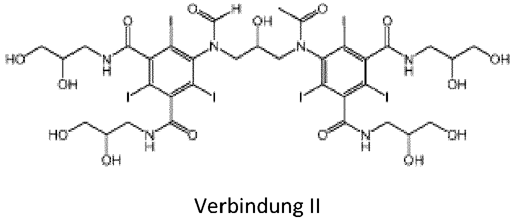

5. Verwendung eines Inhibitors der primären Keimbildung oder des Kristallwachstums in einem Verfahren zur Erhöhung der Stabilität einer Zusammen-setzung, die loforminol umfasst, durch Einschließen des Inhibitors, der eine nichtionische iodierte dimere Verbindung der Formel (I) umfasst, in die Zusammensetzung
R-N(R6)-X-N(R6)-R Formel (I)
wobei
X einen geraden oder verzweigten C3- bis C8-Alkylenteil bezeichnet, wobei optional ein oder zwei CH₂-Teile durch Sauerstoffatome, Schwefelatome oder NR4-Gruppen ersetzt sind und wobei der Alkylenteil optional durch bis zu sechs -OR4-Gruppen substituiert ist;
R4 ein Wasserstoffatom oder eine gerade oder verzweigte C1- bis C4-Alkylgruppe bezeichnet;
R6 ein Wasserstoffatom oder eine Acylfunktion bezeichnet; und
jedes R unabhängig voneinander gleich oder verschieden ist und eine 2,4,6-triiodierte Phenylgruppe bezeichnet, weiter substituiert durch zwei Gruppen R5, wobei jede R5 gleich oder verschieden ist und ein Wasserstoffatom oder einen nichtionischen hydrophilen Teil bezeichnet, vorausgesetzt, dass mindestens eine R5-Gruppe ein hydrophiler Teil ist.

6. Verwendung eines Inhibitors der primären Keimbildung oder des Kristallwachstums in einem Verfahren zur Herstellung einer stabilen Zusammensetzung, die das Kontrastmittel loforminol in einem Träger umfasst, wobei das Verfahren einen Schritt des Einschließens des Inhibitors in die Zusammensetzung aufweist und wobei der Inhibitor eine nichtionische iodierte dimere Verbindung der Formel (I) umfasst
R-N(R6)-X-N(R6)-R Formel (I)
wobei
X einen geraden oder verzweigten C3- bis C8-Alkylenteil bezeichnet, wobei optional ein oder zwei CH₂-Teile durch Sauerstoffatome, Schwefelatome oder NR4-Gruppen ersetzt sind und wobei der Alkylenteil optional durch bis zu sechs -OR4-Gruppen substituiert ist;
R4 ein Wasserstoffatom oder eine gerade oder verzweigte C1- bis C4-Alkylgruppe bezeichnet;
R6 ein Wasserstoffatom oder eine Acylfunktion bezeichnet; und
jedes R unabhängig voneinander gleich oder verschieden ist und eine 2,4,6-triiodierte Phenylgruppe bezeichnet, weiter substituiert durch zwei Gruppen R5, wobei jede R5 gleich oder verschieden ist und ein Wasserstoffatom oder einen nichtionischen hydrophilen Teil bezeichnet, vorausgesetzt, dass mindestens eine R5-Gruppe ein hydrophiler Teil ist.

7. Verwendung nach Anspruch 6, wobei das Verfahren die Schritte umfasst von entweder
a) Kombinieren von loforminolpulver und Pulver eines Inhibitors der primären Keimbildung oder des Kristallwachstums zu einem gemischten Pulver;
Lösen des gemischten Pulvers in einem Träger und Mischen und Erwärmen bis zur vollständigen Auflösung; oder
Lösen von loforminolpulver in einem Träger, um eine erste Zusammensetzung bereitzustellen;
Lösen eines Inhibitors der primären Keimbildung oder des Kristallwachstums in einem Träger, um eine zweite Zusammensetzung bereitzustellen;
Mischen der ersten und zweiten Zusammensetzungen und Erhitzen bis zur vollständigen Auflösung;
oder
Erwärmen einer Zusammensatzung von loforminol in einem Träger,
Hinzufügen eines Pulvers eines Inhibitors der primären Keimbildung oder des Kristallwachstums zu der Zusammensetzung von loforminol und Mischen und Erwärmen bis zur vollständigen Auflösung.

8. Verwendung nach Anspruch 6 oder 7, wobei das Verfahren weiter einen der Schritte des Filterns, Verdünnens, Befüllens, Verschließens und Markierens und der Wärmebehandlung nach dem Befüllen umfasst.

9. Verwendung nach einem der Ansprüche 6 bis 8, wobei der Inhibitor eine chemische Struktur aufweist, wobei mindestens ein Element der chemischen Struktur ähnlich oder identisch mit loforminol ist.

10. Verwendung nach einem der Ansprüche 6 bis 9, wobei der Inhibitor die dimere Verbindung lodixanol oder die Verbindung II umfasst

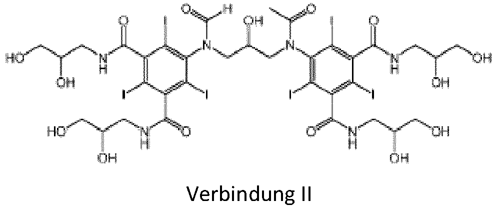

11. Verwendung nach einem der Ansprüche 6 bis 10, wobei der Inhibitor in der Zusammensetzung in einer Menge von 0,1 Gew.-% bis 15 Gew.-% vorhanden ist.

## Revendications

1. Composition comprenant l'agent de contraste ioforminol et comprenant en outre un inhibiteur de nucléation primaire ou de croissance cristalline, dans laquelle cet inhibiteur comprend un composé dimérique iodé non ionique dans lequel l'inhibiteur comprend un composé de formule (I)
R-N(R6)-X-N(R6)-R Formule (I)
dans laquelle
X désigne un fragment alkylène linéaire ou ramifié C3 à C8 facultativement avec un ou deux fragments CH₂ remplacés par des atomes d'oxygène, des atomes de soufre ou des groupes NR4 et dans laquelle le fragment alkylène est facultativement substitué par jusqu'à six groupes -OR4 ;
R4 désigne un atome d'hydrogène ou un groupe alkyle linéaire ou ramifié C1 à C4 ;
R6 désigne un atome d'hydrogène ou une fonction acyle ; et
chaque R indépendamment est identique ou différent et désigne un groupe phényle 2,4,6-triiodé, substitué en outre par deux groupes R5 dans lesquels chaque R5 est identique ou différent et désigne un atome d'hydrogène ou une fraction hydrophile non ionique, à condition qu'au moins un groupe R5 soit une fraction hydrophile.

2. Composition selon la revendication 1, dans laquelle l'inhibiteur est présent dans la composition en une quantité de 0,1 % en poids à 15 % en poids.

3. Composition selon la revendication 1 ou 2, dans laquelle le groupe X désigne une chaîne alkylène choisie parmi du propylène, du butylène et du pentylène substitués par un, deux ou trois groupes hydroxyles.

4. Composition selon l'une quelconque des revendications 1 à 3, dans laquelle l'inhibiteur comprend le composé dimérique iodixanol ou le Composé II

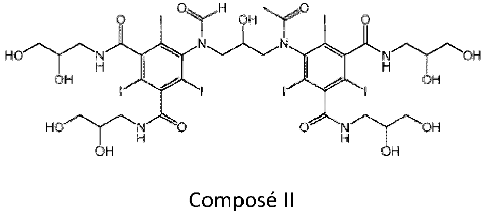

5. Utilisation d'un inhibiteur de nucléation primaire ou de croissance cristalline dans un procédé pour augmenter la stabilité d'une composition comprenant de l'ioforminol, en incluant dans la composition ledit inhibiteur qui comprend un composé dimérique iodé non ionique de formule (I)
R-N(R6)-X-N(R6)-R Formule (I)
dans laquelle
X désigne un fragment alkylène linéaire ou ramifié C3 à C8 facultativement avec un ou deux fragments CH₂ remplacés par des atomes d'oxygène, des atomes de soufre ou des groupes NR4 et dans laquelle le fragment alkylène est facultativement substitué par jusqu'à six groupes -OR4 ;
R4 désigne un atome d'hydrogène ou un groupe alkyle linéaire ou ramifié C1 à C4 ;
R6 désigne un atome d'hydrogène ou une fonction acyle ; et
chaque R indépendamment est identique ou différent et désigne un groupe phényle 2,4,6-triiodé, substitué en outre par deux groupes R5 dans lesquels chaque R5 est identique ou différent et désigne un atome d'hydrogène ou une fraction hydrophile non ionique, à condition qu'au moins un groupe R5 soit une fraction hydrophile.

6. Utilisation d'un inhibiteur de nucléation primaire ou de croissance cristalline dans un procédé de préparation d'une composition stable comprenant l'agent de contraste ioforminol dans un porteur, dans laquelle le procédé inclut une étape d'inclusion dudit inhibiteur dans la composition, et dans laquelle l'inhibiteur comprend un composé dimérique iodé non ionique de formule (I)
R-N(R6)-X-N(R6)-R Formule (I)
dans laquelle
X désigne un fragment alkylène linéaire ou ramifié C3 à C8 facultativement avec un ou deux fragments CH₂ remplacés par des atomes d'oxygène, des atomes de soufre ou des groupes NR4 et dans laquelle le fragment alkylène est facultativement substitué par jusqu'à six groupes -OR4 ;
R4 désigne un atome d'hydrogène ou un groupe alkyle linéaire ou ramifié C1 à C4 ;
R6 désigne un atome d'hydrogène ou une fonction acyle ; et chaque R indépendamment est identique ou différent et désigne un groupe phényle 2,4,6-triiodé, substitué en outre par deux groupes R5 dans lesquels chaque R5 est identique ou différent et désigne un atome d'hydrogène ou une fraction hydrophile non ionique, à condition qu'au moins un groupe R5 soit une fraction hydrophile.

7. Utilisation selon la revendication 6, dans laquelle ledit procédé comprend les étapes consistant à soit
a) combiner de la poudre d'ioforminol et de la poudre d'un inhibiteur de nucléation primaire ou de croissance cristalline en une poudre mélangée ;
dissoudre la poudre mélangée dans un porteur et mélanger et chauffer jusqu'à dissolution complète ;
ou
dissoudre de la poudre d'ioforminol dans un porteur pour fournir une première composition ;
dissoudre un inhibiteur de nucléation primaire ou de croissance cristalline dans un porteur pour fournir une seconde composition ;
mélanger les première et seconde compositions et chauffer jusqu'à dissolution complète ;
ou
chauffer une composition d'ioforminol dans un porteur,
ajouter une poudre d'un inhibiteur de la nucléation primaire ou de croissance cristalline à la composition d'ioforminol et mélanger et chauffer jusqu'à dissolution complète.

8. Utilisation selon la revendication 6 ou 7, dans laquelle ledit procédé comprend en outre l'une quelconque des étapes de filtration, dilution, remplissage, encapsulage et étiquetage, et traitement thermique après remplissage.

9. Utilisation selon l'une quelconque des revendications 6 à 8, dans laquelle ledit inhibiteur a une structure chimique dans laquelle au moins un élément de la structure chimique est similaire ou identique à l'ioforminol.

10. Utilisation selon l'une quelconque des revendications 6 à 9, dans laquelle ledit inhibiteur comprend le composé dimérique iodixanol ou le Composé II

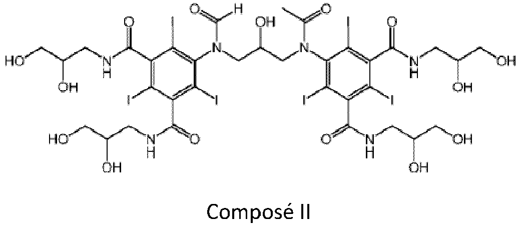

11. Utilisation selon l'une quelconque des revendications 6 à 10, dans laquelle ledit inhibiteur est présent dans la composition en une quantité de 0,1 % en poids à 15 % en poids.
